(19) **Europäisches Patentamt**
**European Patent Office**
**Office européen des brevets**

(11) **EP 3 528 263 A1**

(12) # EUROPEAN PATENT APPLICATION

(43) Date of publication:
**21.08.2019 Bulletin 2019/34**

(51) Int Cl.:
***G16H 50/50*** (2018.01)

(21) Application number: **18156984.9**

(22) Date of filing: **15.02.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Siemens Healthcare GmbH 91052 Erlangen (DE)**

(72) Inventors:
• **Bagrecha, Nitin 90402 Nuremberg (DE)**
• **Brown, Alicia Milford, 48381 (US)**

(54) **PROVIDING A TRAINED VIRTUAL TISSUE ATLAS AND A SYNTHETIC IMAGE**

(57) The invention relates to a method for providing a trained virtual tissue atlas, comprising the step of receiving raw imaging data with an interface, wherein the raw imaging data is based on an imaging procedure of a tissue composition with a medical imaging apparatus; furthermore comprising the step of receiving a tissue parameter of the tissue composition with the interface; furthermore comprising the step of determining a synthetic imaging dataset by applying a neural network onto the raw imaging data with a calculation unit; furthermore comprising the step of adjusting at least one parameter of the neural network based on a comparison of the synthetic imaging dataset and the tissue parameter with the calculation unit; and furthermore comprising the step of providing the trained virtual tissue atlas with the interface, wherein the trained virtual tissue atlas comprises at least the neural network assigned to the tissue parameter.

Furthermore the invention relates to a method for providing a synthetic imaging dataset, comprising the step of receiving a tissue parameter with an interface; furthermore comprising the step of determining a selected neural network stored in a trained virtual tissue atlas with a calculation unit, wherein the trained virtual tissue atlas comprises a plurality of neural networks, each assigned to one of a plurality of tissue parameters; furthermore comprising the step of determining the synthetic imaging dataset based on the selected neural network with the calculation unit, wherein the synthetic imaging dataset is related to the result of imaging a tissue composition related to said tissue parameter with the medical imaging apparatus; and furthermore comprising the step of providing the synthetic imaging dataset with the interface.

FIG 2

## Description

Medical imaging systems, such as magnetic resonance (an acronym is "MR") imaging, require the use of reference objects known as phantoms for calibration and/or quality assurance. Phantoms are also used routinely in imaging training, research and development. As reference objects, phantoms must precisely simulate different kinds of living human tissue, for example fibrous connective tissue. The reliable creation of physical phantoms is known to be complicated. Many are unstable, some may even be hazardous. It is well known that this complex inconsistency and lack of standardization is costly and risky for hospitals, technicians and especially patients.

Physical phantoms are physical objects, often spheres or cylinders, combining a variety of components to match the respective physical characteristics of the human tissue they mimic. It is known to use phantoms comprising water and/or gel (for example agarose, carrageenan or a mixture containing polyvinyl alcohol). Phantoms may comprise a combination of filaments, microparticles, plastics or plastic shapes. Furthermore from the patent application document US 20100202001 A1 it is known to use 3D printing techniques for creating phantoms.

Since stable phantoms are essential for proper quality assurance and the rigors of research in medicine, it is beneficial to use digital phantoms instead of physical phantoms. For example, from the patent document US 7983735 B2 it is known to create a digital phantom model with a pharmacokinetic model to simulate nuclear medical imaging.

So it is the problem of the present invention to provide a digital phantom which relates to an imaging of an arbitrary combination of human tissue.

The problem is solved by a method for providing a trained virtual tissue atlas according to claim 1, a method for providing a synthetic imaging dataset according to claim 4, a providing unit for providing a trained virtual tissue atlas according to claim 10, a providing unit for providing a synthetic imaging dataset according to claim 12, a computer program product according to claim 14, and a computer-readable medium according to claim 15.

In the following the solution according to the invention is described with respect to the claimed providing systems as well as with respect to the claimed methods. Features, advantages or alternative embodiments herein can be assigned to the other claimed objects and vice versa. In other words, claims for the providing systems can be improved with features described or claimed in the context of the methods. In this case, the functional features of the method are embodied by objective units of the providing system.

In one aspect the method relates to a method for providing a trained virtual tissue atlas, comprising the step of receiving raw imaging data with an interface, wherein the raw imaging data is based on an imaging procedure of a tissue composition with a medical imaging apparatus; furthermore comprising the step of receiving a tissue parameter of the tissue composition with the interface; furthermore comprising the step of determining a synthetic imaging dataset by applying a neural network onto the raw imaging data with a calculation unit; furthermore comprising the step of determining a synthetic tissue parameter with the calculation unit; furthermore comprising the step of adjusting at least one parameter of the neural network based on a comparison of the synthetic tissue parameter and the tissue parameter with the calculation unit; and furthermore comprising the step of providing the trained virtual tissue atlas with the interface, wherein the trained virtual tissue atlas comprises at least the neural network assigned to the tissue parameter.

In particular the neural network is assigned to the tissue parameter if the trained virtual tissue atlas comprises a pair comprising the tissue parameter and the neural network. In particular the trained virtual tissue atlas can comprise a list of tissue parameters, wherein each of the tissue parameters is linked to a neural network, wherein said neural network was trained with raw imaging data based on imaging procedures of tissue compositions according to said tissue parameter.

The inventors recognized that the trained virtual tissue atlas provided by this method is suited for system calibration, quality assurance, education, training, research, development and deep learning. Furthermore, by this method the trained virtual tissue atlas can be determined in an efficient way.

According to a further aspect of the invention the tissue composition comprises at least one tissue component, a tissue component being loose connective tissue, fibrous connective tissue, adipose tissue, cartilage, bone and/or blood, and wherein the tissue parameter is based on the ratio of the tissue components in the tissue composition. The inventors have recognized that a tissue parameter based on the ratio of the tissue components describes a tissue composition in a comprehensive but still exact way, leading to a significant reduction of the possible input parameters and to a more comprehensive tissue atlas.

According to a further aspect of the invention the tissue components of the tissue compositions are furthermore discriminated into healthy tissue components and diseased tissue components. The inventors have recognized that the discrimination into healthy and diseased tissue components enables the trained virtual tissue atlas to model tissue components more exactly and specifically.

In another aspect the invention relates to a method for providing a synthetic imaging dataset, comprising the step of receiving a tissue parameter with an interface; furthermore comprising the step of determining a selected neural network stored in a trained virtual tissue atlas with a calculation unit, wherein the trained virtual tissue atlas comprises a plurality of neural networks, each assigned to one of a plurality of tissue parameters;

furthermore comprising the step of determining the synthetic imaging dataset based on the selected neural network with the calculation unit, wherein the synthetic imaging dataset is related to the result of imaging a tissue composition related to said tissue parameter with the medical imaging apparatus; and furthermore comprising the step of providing the synthetic imaging dataset with the interface.

The inventors have recognized that using the selected neural network from a trained virtual tissue atlas allows creating a synthetic imaging dataset that corresponds to an imaging dataset of tissue with the received tissue parameter. The created synthetic imaging dataset can then be used for calibration of the medical imaging apparatus, for optimizing imaging parameters and for training purposes.

According to a further aspect of the invention the method for providing a synthetic imaging dataset furthermore comprises the step of receiving a first imaging parameter of a medical imaging apparatus with the interface, furthermore the step of determining the synthetic imaging dataset is furthermore based on the first imaging parameter. The inventors recognized that determining the synthetic imaging dataset based on the first imaging parameter can improve the quality of the synthetic imaging dataset.

According to a further aspect the method for providing a synthetic imaging dataset furthermore comprises the step of determining a second imaging parameter of the medical imaging apparatus based on the synthetic imaging dataset and the first imaging parameter with the calculation unit. The inventors have recognized that the determined second imaging parameter can take into account the synthetic imaging dataset corresponding to the first imaging parameter, so that the second imaging parameter can be optimized in order to give better imaging results than a usage of the first parameter.

According to a further aspect the method for providing a synthetic imaging dataset furthermore comprises the step of validating the synthetic imaging dataset based on a predictive mechanism with the calculation unit. The inventors have recognized that using a predictive mechanism the synthetic imaging dataset can be provided more exactly and with fewer errors.

According to a further aspect the medical imaging apparatus is a magnetic resonance imaging apparatus. The inventors have recognized that imaging using a magnetic resonance imaging apparatus involves the determination and/or calibration of a lot of imaging parameters, so that by applying the method to a magnetic resonance imaging apparatus in particular leads to a optimization of the imaging procedures.

According to a further aspect of the invention the first imaging parameter and/or the second imaging parameter relate to one of the following parameters: repetition time of a pulse sequence, echo time of a pulse sequence, strength of the homogeneous main magnet field, strength of the inhomogeneous gradient magnet field, flip angle, inversion time of an inversion recovery sequence, and/or imaging slice thickness

In another aspect the invention relates to a providing unit for providing a trained virtual tissue atlas, comprising the following units:

- interface, configured for receiving raw imaging data, wherein the raw imaging data is based on an imaging procedure of a tissue composition with a medical imaging apparatus, furthermore configured for receiving a tissue parameter of the tissue composition, furthermore configured for providing a trained virtual tissue atlas, wherein the trained virtual tissue atlas comprises at least a neural network assigned to a tissue parameter, wherein the tissue parameter is based on the tissue composition;
- calculation unit, configured for determining a synthetic imaging dataset by applying the neural network onto the raw imaging data, furthermore configured for determining a synthetic tissue parameter, furthermore configured for adjusting at least one parameter of the neural network based on a comparison of the synthetic tissue parameter and the tissue parameter.

In particular the providing unit for providing a trained virtual tissue atlas can be configured to execute the method for providing a trained virtual tissue atlas according to the invention and its aspects. The providing system is configured to execute the method and its aspects by the interface and the calculating unit being configured to execute the respective method steps.

In another aspect the invention relates to a providing unit for providing a synthetic imaging dataset, comprising the following units:

- interface, configured for receiving a tissue parameter, furthermore configured for providing the synthetic imaging dataset with the interface;
- calculation unit, configured for determining a selected neural network stored in a trained virtual tissue atlas, wherein the trained virtual tissue atlas comprises a plurality of neural networks, each assigned to one of a plurality of tissue parameters, furthermore configured for determining the synthetic imaging dataset based on the selected neural network with the calculation unit, wherein the synthetic imaging dataset is related to the result of imaging a tissue composition related to said tissue parameter with the medical imaging apparatus.

In particular the providing unit for providing a synthetic imaging dataset can be configured to execute the method for providing a synthetic imaging dataset according to the invention and its aspects. The providing system is configured to execute the method and its aspects by the interface and the calculating unit being configured to execute the respective method steps.

Both providing units can be realized as a data processing system or as a part of a data processing system. Such a data processing system can, for example, comprise a cloud-computing system, a computer network, a computer, a tablet computer, a smartphone or the like. The providing units can comprise hardware and/or software. The hardware can be, for example, a processor system, a memory system and combinations thereof. The hardware can be configurable by the software and/or be operable by the software.

In another aspect the invention relates to a computer program product comprising a computer program, the computer program being loadable into a storage unit of a providing unit, including program code sections to make one of the providing units execute the method for providing a trained virtual tissue atlas or the method for providing a synthetic imaging dataset according to an aspect of the invention when the computer program is executed in said providing unit.

In another aspect the invention relates to a computer-readable medium, on which program code sections of a computer program are saved, said program code sections being loadable into and/or executable in a providing unit to make the providing unit execute the method for providing a trained virtual tissue atlas or the method for providing a synthetic imaging dataset according to an aspect of the invention when the program code sections are executed in providing unit.

The realization of the invention by a computer program product and/or a computer-readable medium has the advantage that already existing providing systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

A medical imaging apparatus is an imaging apparatus for medical purposes. A medical imaging apparatus in particular may relate on imaging using optical electromagnetic radiation and/or X-Ray radiation. In particular, a medical imaging apparatus may be a magnetic resonance (a short term is "MR") imaging apparatus, a computed tomography (a short term is "CT") imaging apparatus, an X-ray imaging apparatus, a positron emission tomography (a short term is "PET") imaging apparatus a C-arm imaging apparatus and/or an ultrasound imaging apparatus.

An imaging dataset comprises one or more images, in particular a two-dimensional image, a three-dimensional image and/or a four-dimensional image. In particular, one of the image dimensions may be a temporal dimension. In particular, all but one or all image dimensions may be spatial dimensions. In particular, an image comprises pixels and/or voxels, wherein to one pixel and/or voxel one or multiple intensity values are assigned. In particular, the one or multiple intensity values correspond to physical parameters of the image region they correspond to.

A synthetic imaging dataset is an imaging dataset being the output of a neural network, in particular if raw imaging data is used as an input for the neural network.

In general raw imaging data is a dataset corresponding to the output of a medical imaging apparatus, wherein the raw imaging data can be used in a step of reconstruction to determine a medical image. In particular, raw imaging data can comprise measurement values taken by one or several sensors of the medical imaging apparatus. Alternatively, raw imaging data can also be synthetically generated, for example by means of a numerical simulation. The term "raw data" can be used as a synonym for "raw imaging data".

A tissue composition is a composition of different types of tissue. In particular, for the description of a tissue composition a tissue component can be classified as healthy tissue or as diseased tissue. In general a tissue parameter corresponds to the structure of a tissue composition, in particular to the ration of different tissue components in a tissue composition. A tissue parameter can be determined based on a measuring or determining the structure of a certain tissue composition, alternatively a tissue parameter can be chosen arbitrarily. In general, a synthetic tissue parameter is the tissue parameter of a synthetic imaging dataset.

In particular, a neural network is based on a collection of nodes, where some of the nodes are interconnected by edges or connections, in particular by directed edges or connections. These edges or connections define the data flow between the nodes of the network. In particular, the edges or connections are equipped with a parameter, wherein the parameter is often denoted as "weight". This parameter can regulate the importance of the output of a first node to the input of a second node, wherein the first node and the second node are connected by an edge. In particular, a neural network can be trained. In particular, training of a neural network is performed based on known pairs of input and output values, wherein the known input values are used as inputs of the neural network, and wherein the corresponding output value of the neural network is compared with the corresponding known output value. The terms "neural network" and "artificial neural network" can be used as synonyms.

The properties, features and advantages of this invention described above, as well as the manner they are achieved, become clearer and more understandable in the light of the following description and embodiments, which will be described in details in the context of the drawings. This following description does not limit the invention on the contained embodiments. Same components or parts can be labeled with the same reference signs in different figures. In general the figures are not for scale. In the following:

Fig. 1   displays different types of human tissue,

Fig. 2   displays a flowchart of a first embodiment of the method for providing a trained virtual tissue atlas,

Fig. 3   displays a flowchart of a second embodiment of the method for providing a trained virtual tissue atlas,

Fig. 4   displays a neural network contained in a trained virtual tissue atlas,

Fig. 5   displays a flowchart of a first embodiment of the method for providing a synthetic imaging dataset,

Fig. 6   displays a flowchart of a second embodiment of the method for providing a synthetic imaging dataset,

Fig. 7   displays a providing unit in a cloud environment.

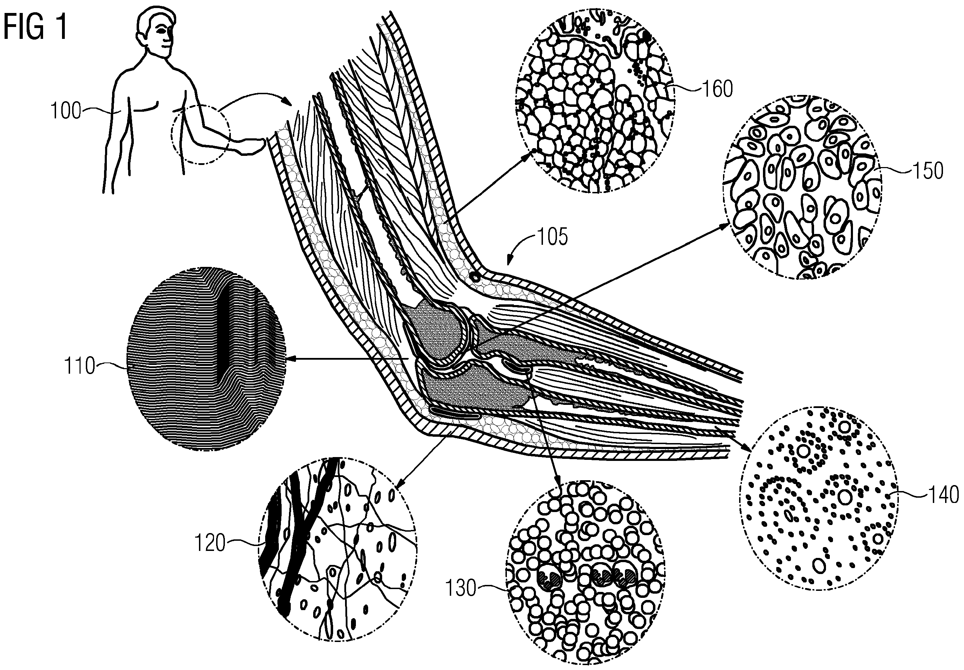

**Fig. 1** displays different types of human tissue of a patient 100, within an example of an arm 105 of the patient 100. Every human tissue has different characteristics, in particular if imaging said human tissue. These characteristics generate different signals in imaging systems. For example, fibrous connective tissue 110 (comprising collagen fibers) that forms tendons, reads differently than loose connective tissue 120 (comprising collagen fibers and elastic fibers) beneath skin. Other examples for tissue types are blood 130 comprising red blood cells, white blood cells and plasma, bone 140 comprising bone forming cells and central canals, cartilage 150 comprising cartilage forming cells embedded in a matrix, and adipose tissue 160 comprising fat droplets. The acquisition of medical images for each tissue type does not differ; in particular the acquisition can be done by well-known methods of interfacing through DICOM to capture raw imaging data.

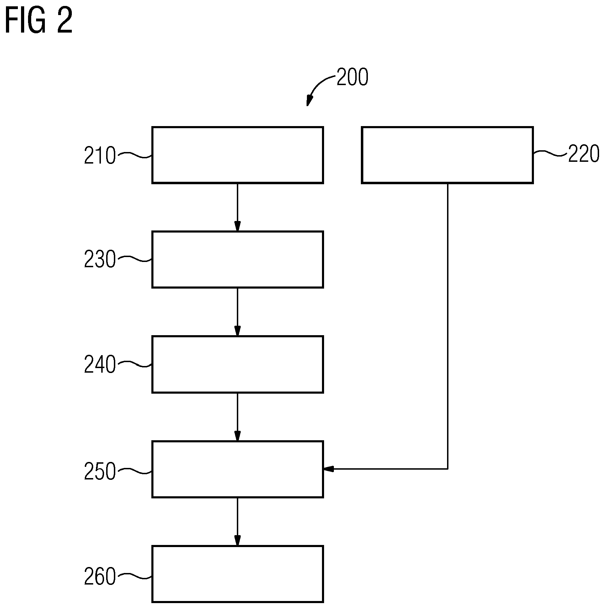

**Fig. 2** displays a flowchart of a first embodiment 200 of the method for providing a trained virtual tissue atlas.

The first step of the displayed first embodiment 200 is receiving 210 raw imaging data with an interface 711, wherein the raw imaging data is based on an imaging procedure of a tissue composition with a medical imaging apparatus 740.

In this embodiment, the medical imaging apparatus 740 is a magnetic resonance tomography apparatus, and the tissue composition comprises 50% of fibrous connective tissue 110 and 50% of loose connective tissue 120.

In particular the raw imaging data of the magnetic resonance tomography apparatus comprises "Mx" and "My" data sampled and stored during data acquisition as a function of time and phase from an MRI sequence. In particular, "Mx" (also called horizontal axis) is the frequency code, and "My" (also called the vertical axis) is the phase code. The raw imaging data can be structured as a raw data matrix, wherein every point within the raw data matrix can comprise information of the complete reconstructed image. In particular and in this example, the raw imaging data can be k-space data.

The next step of the displayed first embodiment 200 is receiving 220 a tissue parameter of the tissue composition with the interface 711. The step of receiving 220 a tissue parameter of the tissue composition can be executed before or after the step of receiving 210 the raw imaging data with the interface 711. Alternatively, the step of receiving 220 a tissue parameter of the tissue composition can be executed in parallel to the step of receiving 210 the raw imaging data with the interface 711. In other words, the step of receiving 220 a tissue parameter of the tissue composition and the step of receiving 210 the raw imaging data are independent.

In this embodiment, the tissue parameter is a vector with six components, each component taking real values between 0 and 1, the sum of all six components being smaller or equal to 1, in particular equal to 1, wherein the first component of the vector corresponds to the ratio of fibrous connective tissue 110 within the tissue composition wherein the second component of the vector corresponds to the ratio of loose connective tissue 120 within the tissue composition, wherein the third component of the vector corresponds to the ratio of blood 130 within the tissue composition, wherein the fourth component of the vector corresponds to the ratio of fibrous bone 140 within the tissue composition, wherein the fifth component of the vector corresponds to the ratio of cartilage 150 within the tissue composition, and wherein the sixth component of the vector corresponds to the ratio of adipose tissue 160 within the tissue composition.

In this example, the tissue composition comprises 50% of fibrous connective tissue 110 and 50% of loose connective tissue 120, which means that the tissue parameter is (0.5, 0.5, 0, 0, 0, 0).

The next step of the displayed first embodiment 200 is determining 230 a synthetic imaging dataset by applying a neural network 400 onto the raw imaging data with a calculation unit 712.

In this embodiment, the input data 410 of the neural network 400 is the k-space data of the magnetic resonance tomography apparatus. In particular, the neural network comprises one input node 420 for every data point from the k-space data. In this particular example, applying the neural network 400 onto the raw imaging data is equivalent to calculating the output data 440 of the neural network 400 when the raw imaging data is the input data 410 of the neural network. In particular, the input data 410 is propagated through the neural network 400, in particular through one or more hidden layers 430.

The next step of the displayed first embodiment 200 is determining 240 a synthetic imaging parameter with the calculation unit 712.

In particular, in this embodiment a synthetic tissue parameter is calculated based on the synthetic imaging dataset, wherein the synthetic imaging dataset parameter is a vector with components corresponding to the ratio of a certain tissue in the synthetic imaging dataset. In particular, for each pixel or voxel of the synthetic imaging dataset it can be determined to which of the certain tissues the pixel or voxel corresponds, for example based on the values assigned to said pixel or voxel or additionally based on the values assigned to neighboring pixels or voxels. In this embodiment, the synthetic tissue parameter is a vector with six components, each component taking real values between 0 and 1, the sum of all six components being smaller or equal to 1, in particular equal to 1, wherein the first component of the vector corresponds to the ratio of fibrous connective tissue 110 within the synthetic imaging dataset, wherein the second component of the vector corresponds to the ratio of loose connective tissue 120 within the synthetic imaging dataset, wherein the third component of the vector corresponds to the ratio of blood 130 within the synthetic imaging dataset, wherein the fourth component of the vector corresponds to the ratio of fibrous bone 140 within the synthetic imaging dataset, wherein the fifth component of the vector corresponds to the ratio of cartilage 150 within the synthetic imaging dataset, and wherein the sixth component of the vector corresponds to the ratio of adipose tissue 160 within the synthetic imaging dataset. In particular, each entry of the synthetic tissue parameter can correspond to an output of the neural network 400. In this embodiment, the output of the neural network 400 is the synthetic imaging dataset, and the synthetic tissue parameter can be calculated based on the synthetic imaging dataset independently from the neural network 400, for example based on another neural network.

Alternatively, the synthetic tissue parameter 440 can also be the output of the neural network 400, and the synthetic imaging dataset corresponds to one of the hidden layers 430. In other words, the neural network 400 calculates the synthetic imaging parameter based on the synthetic imaging dataset. Advantageously the neural network 400 can be pre-trained based on training datasets comprising input raw imaging data and output imaging datasets, wherein the neural network 400 is pre-trained only to the hidden layer 430 corresponding to the synthetic imaging dataset, and wherein the pre-training is based on a comparison of the output imaging dataset and the synthetic imaging dataset.

The next step of the displayed first embodiment 200 is adjusting 250 at least one parameter of the neural network 400 based on a comparison of the synthetic imaging dataset and the tissue parameter with the calculation unit 712. In the displayed embodiment, the at least one parameter of the neural network 400 is an edge weight of the neural network 400.

In particular, in the step of adjusting 250, all weights of the neural network 400 are adjusted. The adjustment of the weights is based on a comparison of the synthetic tissue parameter and the tissue parameter.

In this embodiment the weights of the neural network are adjusted based on the backpropagation algorithm. For example, the synthetic tissue parameter can be the vector (0.4, 0.4, 0.05, 0.05, 0.05, 0.05), the tissue parameter is the vecctor (0.5, 0.5, 0, 0, 0, 0), and the backpropagation is used to minimize the mean squared deviation as parameter, which is in this case equal to 0.03. In this embodiment, the synthetic imaging parameter is calculated based on the synthetic imaging dataset outside of the neural network 400, which implies that the deviation between the synthetic tissue parameter and the tissue parameter needs to be backpropagated through the algorithm that calculates the synthetic imaging parameter based on the synthetic imaging dataset.

In particular, the steps of receiving 210 raw imaging data, the step of receiving 220 a tissue parameter of the tissue composition, the step of determining 230 a synthetic imaging dataset, the step of determining 240 a synthetic tissue parameter and the step of adjusting 250 at least one parameter of the neural network 400 can be executed multiple times, for different sets of raw data corresponding to the same tissue parameter. Alternatively, these steps can also be executed multiple times for different sets of raw data corresponding to different tissue parameters. In particular, said steps can be executed until an error parameter, in particular the error backpropagated, in particular the mean squared error, is below a certain threshold. Furthermore, instead of considering the error parameter of one of the plurality of said training steps, one can also consider a mean error parameter based on several of the plurality of said training steps.

The last step of the displayed first embodiment 200 is providing 260 the trained virtual tissue atlas with the interface 711, wherein the trained virtual tissue atlas comprises at least the neural network 400 assigned to the tissue parameter. In particular, the trained virtual atlas can comprise pairs comprising tissue parameters corresponding to neural networks 400, so that the each of said neural networks 400 are assigned to one of said tissue parameters.

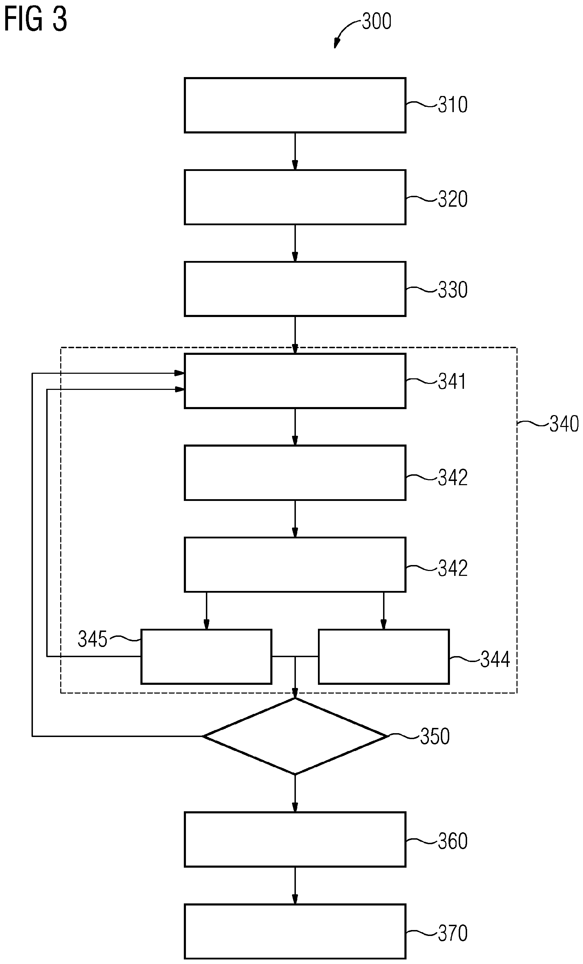

**Fig. 3** displays a flowchart of a second embodiment 300 of the method for providing a trained virtual tissue atlas.

The first step of the second embodiment 300 is receiving 310 a tissue parameter as input with the interface 711. In this embodiment, the tissue parameter is based upon the known variance and features among tissue types and is used to detect the subtle differences between tissues. Live patient or physical phantom data sets for known tissue types can be used. These methods may include real-time acquisition and acquisition from known libraries of identifiable data sets for one or all tissue type.

The second step of the second embodiment 300 is acquiring 320 the raw imaging data with a medical imaging apparatus. In particular, the raw imaging data acquired with the medical imaging apparatus can be received with the interface 711. In particular, before the step of acquiring the raw imaging data the medical imaging apparatus can be calibrated, e.g. by using a physical phantom.

The third step of the second embodiment 300 is preprocessing 330 the raw imaging data with the medical imaging apparatus or with the calculation unit 712. The step of preprocessing can comprise removing outliers of the raw imaging data and/or cleaning the raw imaging data.

The fourth step of the second embodiment 300 is training 340 the artificial neural network 400 with the calculation unit 712. In this second embodiment 300, the step of training 340 comprises the substep of determining 341 a synthetic imaging dataset, the substep of adjusting 342 parameters of the artificial neural network 400 based on the synthetic imaging dataset, the substep of synthesizing 343 a synthetic tissue parameter, the substep of adjusting 344 parameters of the artificial neural network 400 based on the synthetic tissue parameter, and the substep of comparative learning 345. All substeps are executed by the calculation unit 712.

In particular, it is possible that only one substeps of the group of substep of adjusting 344 parameters of the artificial neural network 400 based on the synthetic tissue parameter and the substep of comparative learning 345 is executed. In other words, these substeps can be considered as alternatives.

The purposeful outcome of the training 340 of the artificial neural network 400 is to reconstruct reusable tissues as an atlas available for medical imaging systems. To achieve this outcome the system performs reiterative action. It is programmed fundamentally to acquire a data set from tomographic images, use the data to segment tissues from image slices, assign tissues with a unique identifier, identify and specify aspects of the chemistry and density of the tissues, and register the segments as data for use by the requesting imaging system.

In this second embodiment 300 the synthesizing 343 of the synthetic tissue parameter is based on the synthetic imaging dataset, wherein the synthetic imaging dataset is the output of the artificial neural network 400. In other words, the synthetic tissue parameter is not an output of the artificial neural network 400, but calculated independently by means of calculation unit 712. Alternatively, the synthetic tissue parameter may be an output of the artificial neural network 400; in particular, in this case the synthetic imaging dataset can be the output of a hidden layer 430 of the artificial neural network 400.

In this second embodiment 300, there are two substeps of learning, the first substep is the substep of adjusting 342 parameters of the artificial neural network 400 based on the synthetic imaging dataset, the second substep is the substep of adjusting 344 parameters of the artificial neural network 400 based on the synthetic tissue parameter. Since there is no direct comparison value within the training data for the synthetic imaging dataset, adjusting 342 parameters of the artificial neural network 400 based on the synthetic imaging dataset cannot rely on the direct comparison of the synthetic imaging dataset with a training image. So in the second embodiment 300 adjusting 342 parameters of the artificial neural network 400 is based on a quality parameter of the synthetic imaging dataset, wherein the quality parameter can be based on the sharpness, the noise, the dynamic range, the contrast, or artifacts contained in the synthetic imaging dataset. For the substep of adjusting 344 parameters of the artificial neural network 400 based on the synthetic tissue parameter the synthetic tissue parameter can be compared with the tissue parameter used as an input, which is the real tissue parameter of the sample. In this embodiment, adjusting 344 parameters of the artificial neural network 400 is done by using the backpropagation algorithm twice, firstly on the (known or given) method for calculating the tissue parameter based on the synthetic imaging dataset, and secondly on the artificial neural network 400 calculating the synthetic imaging dataset based on the raw imaging data.

The displayed second embodiment 300 furthermore comprises the optional step of monitoring and correcting 350 image quality, the step of classifying the image 360 and the step of providing 370 the tissue atlas comprising the artificial neural network 400.

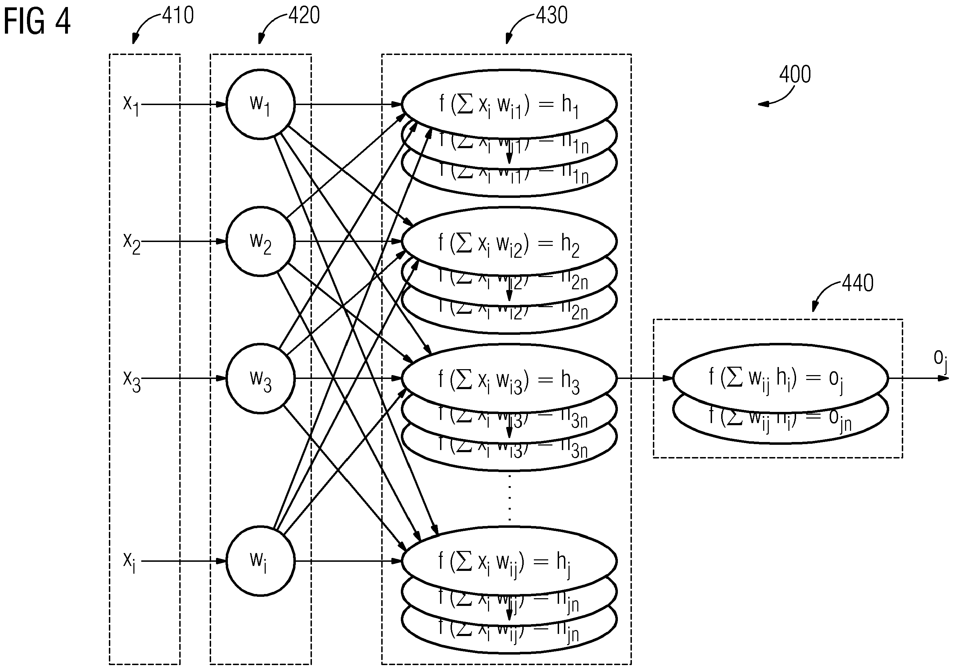

**Fig. 4** displays an artificial neural network 400 used in the method displayed in Fig. 2. In general the artificial neural network 400 responds to the input of multiple input nodes $x_i$ 410 that are applied to generate one or more outputs $o_j$ which may be in multiple iterations and sets of iterations, known as epochs. Learning is performed by the artificial neural network 400 in this embodiment by adapting the weights and biases applied to the neurons based on the training data.

Possible values for the input nodes 410 are the data calibrations, multiple MR volumes, raw image and tissue characteristics, and as above, as well as learned parameters. The present invention is not limited to these inputs as other inputs not discussed herein may be applied. The artificial neural network 400 weighs 420 the inputs 410 based upon the learning process. The output 440 of the artificial neural network 400 is preferably data that forms an image that needs no adjustment. It is also possible that there are several output nodes 440. However, other inputs are likely required as adjustments for the steps of adjusting 250, 342, 344.

The artificial neural network 400 also includes the hidden layer 430 that is made up of multiple neurons $h_j$. There may be multiple hidden layers, as displayed in 430 as $h_{jn}$, and a hidden layer 430 can use the output of another hidden layer 430 as input. The neurons 430 are capable of performing a simple mathematical task. The output of each neuron $h_j$ is a nonlinear function f of its inputs $x_i$ and the weights and biases $w_i$. Upon receiving the inputs $x_i$, the neurons $h_j$ perform a summation of a weighted $w_i$ multiplication of each input $x_i$, as defined by

$$h_j = f\left(\sum_i x_i \cdot w_{ij}\right)$$

In particular, the neural output $h_j$ is calculated as a function f of neuron activation, which may be, for example, a hyperbolic tangent sigmoidal function or a linear ramp function. The neural outputs $h_j$ are applied to the output node $O_j$. A summation of a weighted multiplication of each neural output $h_j$ is calculated as a function of the output node activation.

$$o_j = f\left(\sum_i h_i \cdot w'_{ij}\right)$$

The artificial neural network 400 is a feed-forward neural network in that each neuron 430 processes all of the inputs from a previous layer by accepting the weighted sum of these inputs. It is understood that in this feature learning, other types of neural networks such as feed-back neural networks may be employed as well, where the input of a neuron $h_j$ is also one of that neuron's outputs.

The artificial neural network 400 is trained to recognize patterns using one or more supervised training methods known to those skilled in the art. One such method may be backpropagation, although it, or any method, may be applied in accordance with one or more embodiments of the invention. During training, the artificial neural network 400 is exposed to training input sets, and generates corresponding outputs. Mean square errors (an acronym is "MSE") are calculated for the artificial neural network 400 between the corresponding outputs and the desired outputs. In this, weight factors 420 may be adjusted in the artificial neural network 400 to minimize the mean square error. This process repeats until the mean square error is below tolerance as it approaches zero and as determined by the methods.

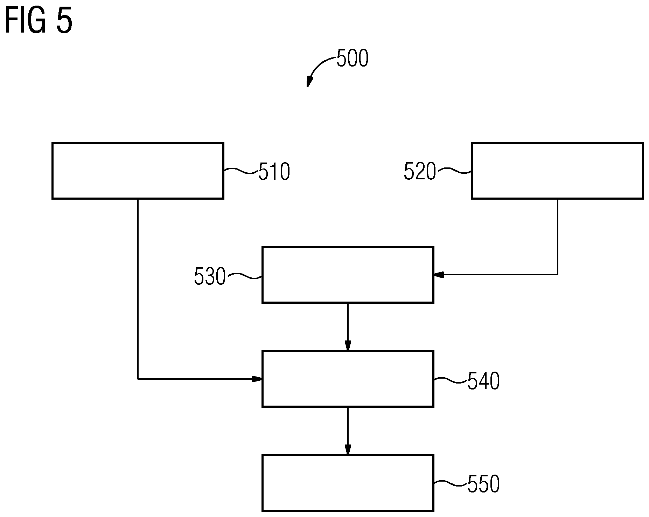

Fig. 5 displays a flowchart of a first embodiment 500 of the method for providing a synthetic imaging dataset.

The first step of the first embodiment 500 receiving 510 a first imaging parameter of a medical imaging apparatus 740 with an interface 711. The step of receiving a first imaging parameter is an optional step. In this first embodiment, the medical imaging apparatus 740 is a magnetic resonance imaging apparatus, and the first imaging parameter comprises details of the imaging procedure with the magnetic resonance imaging apparatus, for example the repetition time of a pulse sequence, the echo time of a pulse sequence, the strength of the homogeneous main magnet field, the strength of the inhomogeneous gradient magnet field, the flip angle, the inversion time of an inversion recovery sequence and/or the imaging slice thickness of medical imaging procedure executed with the magnetic resonance imaging apparatus.

The second step of the first embodiment 500 is receiving 520 a tissue parameter with the interface 711. In this first embodiment 500 the structure of the tissue parameter is equivalent with the structure described for the first embodiment 200 of the method for providing a trained virtual tissue atlas. In this embodiment the tissue parameter being received was a manual input of an operator using the medical imaging apparatus 740, wherein the tissue parameter is an estimation of the tissue parameter of the tissue composition in the field of view of the medical imaging apparatus 740 estimated by the operator. Alternatively, the tissue parameter can be extracted from a database storing tissue parameters for different field of views used in the imaging procedure with the medical imaging apparatus 740 and for different patient parameters (e.g. height, weight and/or age of the patient). The extraction of the tissue parameter can optionally include an interpolation of the tissue parameter, in particular if there is no tissue parameter in the database for a certain combination of patient parameters.

The third step of the first embodiment 500 is determining 520 a selected neural network 400 stored in a trained virtual tissue atlas with a calculation unit 712, wherein the trained virtual tissue atlas comprises a plurality of neural networks, each assigned to one of a plurality of tissue parameters. In this first embodiment the selected neural network 400 is the one of the plurality of neural networks stored in the virtual tissue with a tissue parameter assigned which is closest to the received tissue parameter.

In particular, the assigned tissue parameter is closest to the received tissue parameter, if the result of a distance measure applied to the assigned tissue parameter and the received tissue parameter is minimal compared to an application to other tissue parameters stored in the virtual training atlas and the received tissue parameter. In particular, if the assigned and the received tissue parameter comprise several numerical values, the distance measure can be the mean squared difference of said numerical values. In this embodiment, both the assigned and the received tissue parameters are vectors with six components, each component taking real values between 0 and 1, and the distance measure is the mean squared difference of the six components.

The fourth step of the first embodiment 500 is determining 540 the synthetic imaging dataset based on the selected neural network 400 with the calculation unit 712, wherein the synthetic imaging dataset is related to the result of imaging a tissue composition related to said tissue parameter with the medical imaging apparatus. In particular, in this first embodiment 500 the determining 530 of the synthetic imaging dataset is determined by using synthetic raw imaging data as input to the selected neural network 400, using the output of the selected neural network 400 as synthetic imaging dataset. In particular, the synthetic raw imaging data can be calculated by simulating an image acquisition of a tissue composition having said tissue parameter, e.g. by the use of a Monte-Carlo simulation. In particular, the calculation of the synthetic raw imaging data can furthermore be based on the first imaging parameter, by the first imaging parameter being used as a parameter of the Monte-Carlo simulation.

The fifth step of the first embodiment 500 is providing 550 the synthetic imaging dataset with the interface 711. Alternatively, the synthetic imaging dataset can be displayed by an input and/or output unit 714 of the providing unit 711.

Alternatively and not displayed in Fig. 5 the method for providing 550 the synthetic imaging dataset can comprise a step of determining a second imaging parameter of the medical imaging apparatus based on the synthetic imaging dataset and the first imaging parameter with the calculation unit. In particular, the second imaging parameter can be calculated by modifying the first imaging parameter to improve the synthetic imaging dataset. In particular, multiple synthetic imaging datasets can be calculated based for multiple imaging parameters, and the one imaging parameter inducing the synthetic imaging dataset with an optimal quality parameter can be chosen as second imaging parameter. In particular, the second imaging parameter can be provided interface 711.

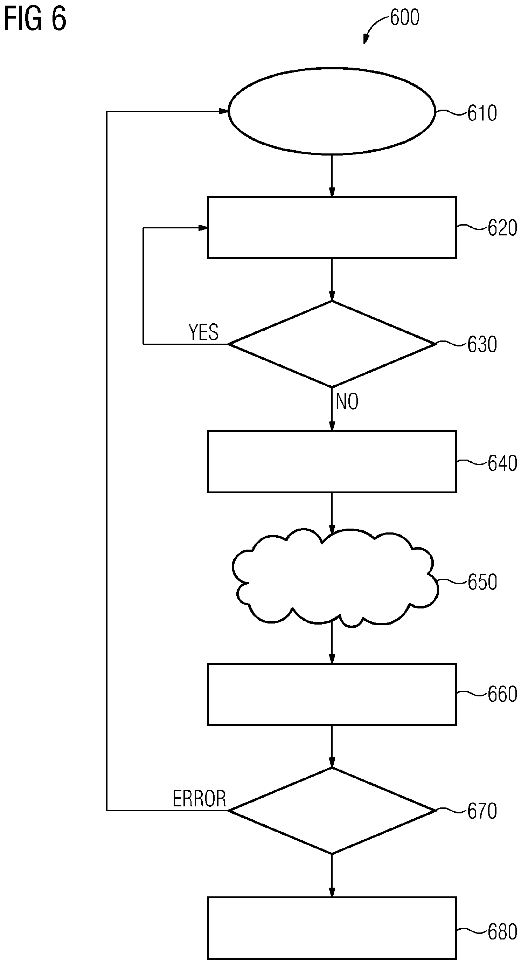

Fig. 6 displays a flowchart of a second embodiment 600 of the method for providing a synthetic imaging dataset. This embodiment may in turn, repeatedly reconstruct any given tissue, single and mixed, healthy and diseased, without restricting the general applicability, and through a cloud-based computer 710, make phantoms of all tissue types in an atlas available for, among other things, system calibration, quality assurance, education, training, research, development and deep learning.

In second embodiment 600 a medical imaging apparatus 740 accesses 610 the virtual tissue atlas real-time through well-known methods for those skilled in the art. Imaging systems communicate with external systems via DICOM standard interchange protocol and may communicate in other protocols and file formats. In this example, following standard DICOM setup and methods, the medical imaging apparatus 740 is a magnetic resonance imaging apparatus and is configured to interface with the system.

A request is sent 610 to the system to access a tissue from the atlas, by providing the received tissue parameter and a first imaging parameter. The system responds as it provides 620 a synthetic imaging dataset based on the received tissue parameter and on first imaging parameters. Within this step of providing 620, the system simulates acquisition effects based on the first imaging parameter, wherein the first imaging parameter may comprise the repetition time of a pulse sequence, the echo time of a pulse sequence, the strength of the homogeneous main magnet field, the strength of the inhomogeneous gradient magnet field, the flip angle, the inversion time of an inversion recovery sequence and/or the imaging slice thickness. The next step is then to validate 630 the response and corrects through a predictive mechanism. The next step of the second embodiment 600 is to share or provide 640 the phantom. The image is sent 650 through a secure internet connection. As a further step, the synthetic imaging dataset is received 660 by the medical imaging apparatus 740. An operator of the medical imaging apparatus 740 has an opportunity to make manual adjustments to the target image and set 670 a second imaging parameter. The phantom is used 680 by the medical imaging apparatus 740 as the target image is aligned.

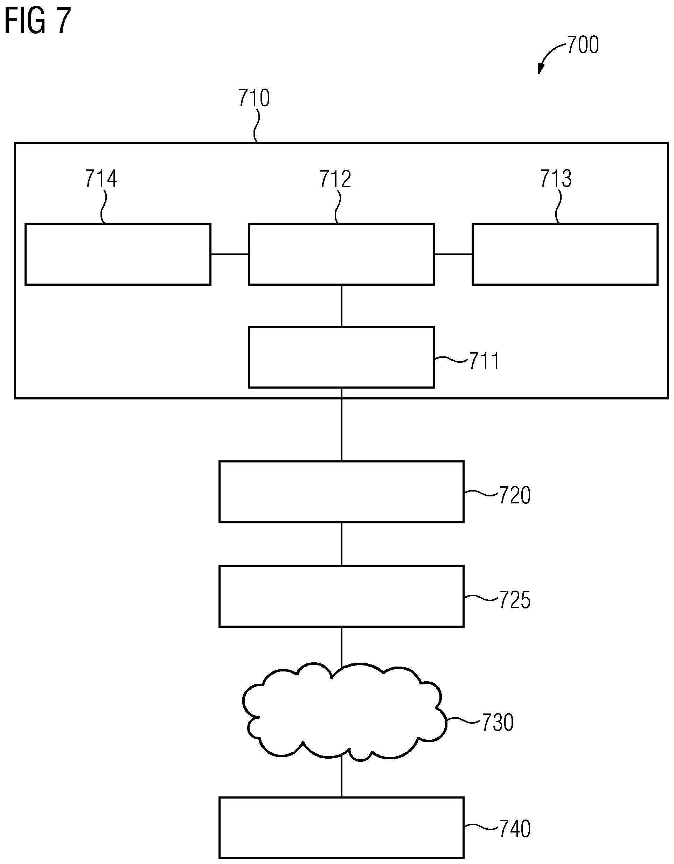

Fig. 7 displays a providing unit 710 within a cloud environment 700. In this embodiment the cloud environment furthermore comprises a DICOM interface 720 and a firewall 725, wherein the DICOM interface 720 and the firewall 725 may comprise hardware and software elements. The system may be available to a medical imaging apparatus 740 that has been configured to access the providing unit 710 by means of a network 730, and by the DICOM interface 720 and the firewall 725.

A providing unit 710 may be a (personal) computer, a workstation, a virtual machine running on host hardware, a microcontroller, or an integrated circuit. As an alternative, the providing unit 710 can be a real or a virtual group of computers (the technical term for a real group of computers is "cluster", the technical term for a virtual group of computers is "cloud"). The providing unit 710 comprises an interface 711, a calculation unit 712, a storage unit 713 and an input/output unit 714.

The providing unit 710 is configured both for executing the methods for providing a trained virtual tissue atlas and the method for providing a synthetic imaging dataset.

An interface 711 can be embodies as a hardware interface or as a software interface (e.g. PCI-Bus, USB or Firewire). In general, a calculation unit 712 can comprise hardware elements and software elements, for example a microprocessor, a field programmable gate array (an acronym is "FPGA") or an application specific integrated circuit (an acronym is "ASIC"). A storage unit 713 can be embodied as non-permanent main memory (e.g. random access memory) or as permanent mass storage (e.g. hard disk, USB stick, SD card, solid state disk). The input/output unit 714 can comprise means for inputting data (e.g. a keyboard or a mouse) and/or means for outputting data (e.g. a display or a printer).

The communication between the providing unit 710 and the medical imaging apparatus 730 is based in DICOM in this embodiment, well-known to those skilled in the art. This standard interchange protocol provides extensive two-way communication with imaging systems.

The network 730 can be realized as a LAN (acronym for "local area network"), in particular a WiFi network, or any other local connection, e.g. via Bluetooth or USB (acronym for "universal serial bus"). The network 730 can also be realized as a WAN (acronym for "wide area network"), in particular the network 730 can be identical with the internet. The network 730 can alternatively also be realized as a VPN (acronym for "virtual private network").

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

**Claims**

1. A method for providing a trained virtual tissue atlas, comprising the following steps:

   - receiving (210, 320) raw imaging data with an interface (711), wherein the raw imaging data is based on an imaging procedure of a tissue composition with a medical imaging apparatus (740);
   - receiving (220, 310) a tissue parameter of the tissue composition with the interface (711);
   - determining (230, 341) a synthetic imaging dataset by applying a neural network (400) onto the raw imaging data with a calculation unit (712);
   - determining (240, 343) a synthetic tissue parameter with the calculation unit (712);
   - adjusting (250, 342, 344) at least one parameter of the neural network (400) based on a comparison of the synthetic tissue parameter and the tissue parameter with the calculation unit (712);
   - providing (260, 370) the trained virtual tissue atlas with the interface (711), wherein the trained virtual tissue atlas comprises at least a neural network (400) assigned to the tissue parameter.

2. The method according to claim 1, wherein the tissue composition comprises at least one tissue component, a tissue component being loose connective tissue, fibrous connective tissue, adipose tissue, cartilage, bone and/or blood, and wherein the tissue parameter is based on the ratio of the tissue components in the tissue composition.

3. The method according to claim 2, wherein at least one tissue component of the tissue compositions is furthermore discriminated into a healthy tissue component and a diseased tissue component.

4. A method for providing a synthetic imaging dataset, comprising the following steps:

   - receiving (520, 610) a tissue parameter with an interface (711);
   - determining (530) a selected neural network stored in a trained virtual tissue atlas based on the tissue parameter with a calculation unit (712), wherein the trained virtual tissue atlas comprises a plurality of neural networks, each assigned to one of a plurality of tissue parameters,
   - determining (540) the synthetic imaging dataset based on the selected neural network with the calculation unit (712), wherein the synthetic imaging dataset is related to the result of imaging a tissue composition related to said tissue parameter with a medical imaging apparatus;
   - providing (550, 620) the synthetic imaging dataset with the interface (711).

5. The method according to claim 4, furthermore comprising the following step:

   - receiving (510, 610) a first imaging parameter of a medical imaging apparatus with the interface (711);
   wherein the step of determining (540) the synthetic imaging dataset is furthermore based on the first imaging parameter.

6. The method according to claim 4 or 5, furthermore comprising the following steps:

   - determining (670) a second imaging parameter of the medical imaging apparatus based on the synthetic imaging dataset and the first imaging parameter with the calculation unit (712).

7. The method according to one of the claims 4 to 6, furthermore comprising the following step:

   - validating (630) the synthetic imaging dataset based on a predictive mechanism with the calculation unit (712).

8. The method according to one of the claims 4 to 7, wherein the medical imaging apparatus (740) is a magnetic resonance imaging apparatus.

9. The method according to claim 8, wherein the first imaging parameter and/or the second imaging parameter relate to one of the following parameters:

   - repetition time of a pulse sequence

- echo time of a pulse sequence
- strength of the homogeneous main magnet field
- strength of the inhomogeneous gradient magnet field
- flip angle
- inversion time of an inversion recovery sequence
- imaging slice thickness

10. A providing unit (710) for providing a trained virtual tissue atlas, comprising the following units:

- interface (711), configured for receiving (210, 320) raw imaging data, wherein the raw imaging data is based on an imaging procedure of a tissue composition with a medical imaging apparatus (740),
furthermore configured for receiving (220, 310) a tissue parameter of the tissue composition,
furthermore configured for providing (260, 370) a trained virtual tissue atlas, wherein the trained virtual tissue atlas comprises at least a neural network (400) assigned to a tissue parameter, wherein the tissue parameter is based on the tissue composition;
- calculation unit (712), configured for determining (230) a synthetic imaging dataset by applying the neural network (400) onto the raw imaging data,
furthermore configured for determining (240, 343) a synthetic tissue parameter,
furthermore configured for adjusting (250, 342, 344) at least one parameter of the neural network (400) based on a comparison of the synthetic tissue parameter and the tissue parameter.

11. The providing unit (710) according to claim 10, furthermore configured to implement the method for providing the trained virtual tissue atlas according to claim 2 or claim 3.

12. A providing unit (710) for providing a synthetic imaging dataset, comprising the following units:

- interface (711), configured for receiving (520, 610) a tissue parameter,
furthermore configured for providing (550, 620) the synthetic imaging dataset with the interface;
- calculation unit (712), configured for determining (530) a selected neural network (400) stored in a trained virtual tissue atlas, wherein the trained virtual tissue atlas comprises a plurality of neural networks, each assigned to one of a plurality of tissue parameters,
furthermore configured for determining (540) the synthetic imaging dataset based on the selected neural network (400), wherein the synthetic imaging dataset is related to the result of imaging a tissue composition related to said tissue parameter with a medical imaging apparatus (740).

13. The providing unit (710) according to claim 12, furthermore configured to implement the method for providing a synthetic imaging dataset according to one of the claims 5 to 9.

14. A computer program product comprising program elements which induce a providing unit (710) to carry out the steps of the method for providing a synthetic imaging dataset according to one of the claims 4 to 9, when the program elements are loaded into a memory (713) of the providing unit (710).

15. A computer-readable medium on which program elements are stored that can be read and executed by a providing unit (710), in order to perform steps of the method for providing a synthetic imaging dataset according to one of the claims 4 to 9, when the program elements are executed by the providing unit (710).

FIG 1

100

105

110

120

130

140

150

160

EP 3 528 263 A1

FIG 2

210

230

240

250

260

200

220

FIG 3

300

310

320

330

341

340

342

342

345

344

350

360

370

FIG 4

$x_1 \rightarrow$ $w_1$ $\rightarrow$ $f\left(\sum x_i w_{i1}\right) = h_1$

$f\left(\sum x_i w_{i1}\right) = h_{1n}$

$f\left(\sum x_i w_{i1}\right) = h_{1n}$

$x_2 \rightarrow$ $w_2$ $\rightarrow$ $f\left(\sum x_i w_{i2}\right) = h_2$

$f\left(\sum x_i w_{i2}\right) = h_{2n}$

$f\left(\sum x_i w_{i2}\right) = h_{2n}$

$x_3 \rightarrow$ $w_3$ $\rightarrow$ $f\left(\sum x_i w_{i3}\right) = h_3$

$f\left(\sum x_i w_{i3}\right) = h_{3n}$

$f\left(\sum x_i w_{i3}\right) = h_{3n}$

$x_i \rightarrow$ $w_i$ $\rightarrow$ $f\left(\sum x_i w_{ij}\right) = h_j$

$f\left(\sum x_i w_{ij}\right) = h_{jn}$

$f\left(\sum x_i w_{ij}\right) = h_{jn}$

$f\left(\sum w_{ij} h_i\right) = o_j$

$f\left(\sum w_{ij} h_i\right) = o_{jn}$

$\rightarrow o_j$

410 420 430 440 400

# FIG 5

FIG 6

FIG 7

**EUROPEAN SEARCH REPORT**

Europäisches Patentamt
European Patent Office
Office européen des brevets

Application Number

EP 18 15 6984

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/021861 A2 (AGNIHOTRI AASHIISH [US]; NADZADI MARK ELLSWORTH [US]; RUMERY MEGAN PAT) 16 February 2012 (2012-02-16) * page 17, line 18 - page 19, line 9; claims; figure 8 * | 1-3 | INV. G16H50/50 |
| X | KAMRAN KAZEMI ET AL: "Design and construction of a brain phantom to simulate neonatal MR images", COMPUTERIZED MEDICAL IMAGING AND GRAPHICS, PERGAMON PRESS, NEW YORK, NY, US, vol. 35, no. 3, 11 November 2010 (2010-11-11), pages 237-250, XP028163637, ISSN: 0895-6111, DOI: 10.1016/J.COMPMEDIMAG.2010.11.007 [retrieved on 2010-11-25] * abstract; figures 5,6 * | 4-15 | |
| X | AUBERT-BROCHE B ET AL: "A new improved version of the realistic digital brain phantom", NEUROIMAGE, ELSEVIER, AMSTERDAM, NL, vol. 32, no. 1, 1 August 2006 (2006-08-01), pages 138-145, XP024906132, ISSN: 1053-8119, DOI: 10.1016/J.NEUROIMAGE.2006.03.052 [retrieved on 2006-08-01] * the whole document * | 4-15 | TECHNICAL FIELDS SEARCHED (IPC) G16H G06K G06T A61B G06F |

The present search report has been drawn up for all claims

| Place of search | Date of completion of the search | Examiner |
|---|---|---|
| Munich | 1 June 2018 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 6984

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2018

| Patent document cited in search report | Publication date | Patent family member(s) | Publication date |
|---|---|---|---|
| WO 2012021861    A2 | 16-02-2012 | CN      103607947  A<br>EP       2603136  A2<br>KR   20160103562  A<br>RU    2013110371  A<br>US    2013336553  A1<br>US    2017200272  A1<br>WO    2012021861  A2<br>WO    2012021862  A2<br>ZA       201301586  B | 26-02-2014<br>19-06-2013<br>02-09-2016<br>20-09-2014<br>19-12-2013<br>13-07-2017<br>16-02-2012<br>16-02-2012<br>27-11-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100202001 A1

- US 7983735 B2